# EUROPEAN PATENT APPLICATION

(11) **EP 2 074 930 A1**
(43) Date of publication of application: **01.07.2009**
(21) Application number: 08022514.7
(22) Date of filing: 29.12.2008
(51) Int. Cl.: A61B 1/005, A61B 1/05

(54) **Electronic endoscope**

(30) Priority: 27.12.2007 JP 2007338056
(71) Applicant: FUJIFILM Corporation, Minato-ku Tokyo (JP)
(72) Inventor: Yashiro, Takashi, Saimtama-shi Saitama (JP); Takahashi, Kazuaki, Saimtama-shi Saitama (JP); Yamamoto, Goki, Saimtama-shi Saitama (JP); Kitano, Ryou, Saimtama-shi Saitama (JP)
(74) Representative: Klunker . Schmitt-Nilson . Hirsch

(57) **Abstract**

A connector (40) includes a cable attachment ring (47) that fits onto a signal cable (24), a circuit board attachment part (48) that catches a circuit board (45), and a connecting pillar (49) that connects the cable attachment ring (47) and the circuit board attachment part (48). The cable attachment ring (47) is detachable from the signal cable (24), and the circuit board attachment part (48) is detachable from the circuit board (45).

## Description

### FIELD OF THE INVENTION

The present invention relates to an electronic endoscope having an imaging unit at a distal end of an insertion section and a signal cable connected to this imaging unit.

### BACKGROUND OF THE INVENTION

Electronic endoscopes (hereinafter, endoscope) are frequently used for medical diagnosis. Typically, the endoscope (or a so-called flexible scope) includes a thin, long and flexible insertion section to be inserted into a patient's body cavity, an operating section connected to a base end of the insertion section, and a universal cord to be connected to a processor unit and a light source unit. In the distal end of the insertion section, an imaging unit having a solid-state imaging element is incorporated. Image light of a region-of-interest (target body part) in the cavity is transmitted through an observation window in the distal end and converted by the imaging unit into an image signal, which is transmitted to the processor unit by way of the operating section and a signal cable that is inserted in the universal cord.

During operation of the endoscope, the insertion section is curved and twisted in all the directions, and the signal cable in the insertion section goes around in a width direction and in a longitudinal direction of the insertion section. This signal cable is generally composed of a plurality of signal lines and an outer jacket to surround these signal lines, which are exposed from a distal end of the signal cable and soldered to a circuit board of the imaging unit. This soldering, however, does not provide adequate strength to the connecting part, and as strong physical load is applied to the connecting part, the solders may be detached and the signal lines may be disconnected.

To avoid this problem, the distal end of the signal cable is attached integrally to the imaging unit by adhesive (see, for example, Japanese Patent Laid-open Publications No. 11-19035 and No. 11-262467). Besides, there is disclosed another approach to connect the signal cable and the imaging unit with a cord, which is then kept strained (see, for example, Japanese Patent Laid-open Publications No. 2007-7179 and No. 2000-107124).

However, the technique to use the adhesive, as disclosed in the Publications No. 11-19035 and No. 11-262467, causes other problems that the positional relationship between the signal cable and the imaging unit cannot be adjusted after assembly, that the signal cable and the imaging unit cannot be repaired in case of trouble, and that care is required to fix them with adhesive.

Also, the technique to use a cord, as disclosed in the Publications No. 2007-7179 and No. 2000-107124, causes the problems that the distance between the signal cable and the imaging unit cannot be adjusted once they are connected with the cord, and that it takes time to wrap the cord. In addition, the cord is resistant to tensile load, but not resistant to torsional load.

### SUMMARY OF THE INVENTION

In view of the foregoing, it is an object of the present invention to provide an electronic endoscope having improved durability in a connecting part between a signal cable and an imaging unit.

Another object of the present invention is to provide an electronic endoscope capable of improving workability during assembly and repair.

In order to achieve the above and other objects, an electronic endoscope according to the present invention includes an insertion section to be inserted into an object under observation, an imaging unit disposed at a front end of the insertion section to image a region-of-interest in the object, a signal cable extending through the insertion section, and a connector for connecting a front end portion of the signal cable and the imaging unit. The signal cable is composed of a plurality of signal lines and a cable jacket which surrounds these signal lines. The signal lines are exposed from a front end of the signal cable and connected to the imaging unit. The connector is made of an electrical insulating material.

In a preferred embodiment of the present invention, the connector includes a cable attachment part to be attached detachably to the front end portion of the signal cable, an imaging unit attachment part to be attached detachably to the imaging unit, and a connecting piece for connecting the cable attachment part and the imaging unit attachment part.

In another preferred embodiment of the present invention, the connector surrounds the exposed part of the signal lines and a plurality of contact terminals which are located in the imaging device and connected to the signal lines. In this case, the connector is preferably made of rubber.

According to the present invention, the signal cable and the imaging unit are coupled with the connector, and the connecting part between them becomes durable enough to endure both tensile load and torsional load. In addition, because of the detachability of the connector, the positional relationship between the signal cable and the imaging unit can be adjusted easily during assembly. In case of trouble, the connector is simply removed, and the signal cable and the imaging unit can easily be repaired.

### BRIEF DESCRIPTION OF THE DRAWINGS

The above objects and advantages of the present invention will become more apparent from the following detailed description when read in connection with the accompanying drawings, in which:
FIG. 1 is an external view of an electronic endoscope according to the present invention;
FIG. 2 is a cross sectional view of a flexible tube;
FIG. 3 is an elevation view of an end surface of an insertion section;
FIG. 4 is an axial cross sectional view of a distal end;
FIG. 5 is an explanatory view illustrating a distal end of a signal cable, a circuit board, and a connector;
FIG. 6 is a perspective view of the connector;
FIG. 7 is a plan view of a swingable connector;
FIG. 8 is a plan view of a connector configured to surround signal lines exposed from the signal cable and input/output terminals of the circuit board; and
FIG. 9 is a perspective view of the connector of FIG. 8.

### DETAILED DESCRIPTION OF THE PREFERRED EMBODIMENTS

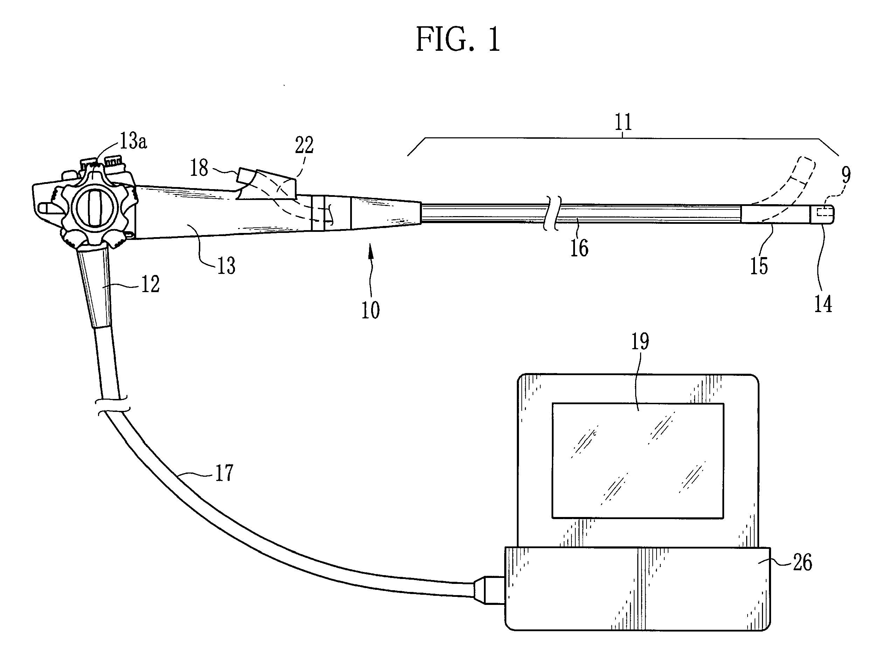

Referring to FIG. 1, an electronic endoscope 10 is a long and thin instrument that includes an insertion section 11, a connecting section 12, and an operating section 13. The insertion section 11 is composed of a front end part 14 on the distal end, a curving part 15 and a flexible tube part 16 that is connected to the operating section 13. The connecting section 12 is connected to a processor unit 26, an air supply unit (not shown) and a light source unit (not shown) through a universal cord 17. The processor unit 26 has an imaging controller that provides a drive signal to an imaging unit 9 in the front end part 14, and an image processor for processing an image signal generated from the imaging unit 9.

The curving part 15 curves left, right, up or down when an angle knob 13a provided on the operating section 13 is rotated to push or pull an angle wire installed in the insertion section 11. The flexible tube part 16 is a long, thin and flexible component that stretches between the operating section 13 and the curving part 15. This configuration allows orienting the front end part 14 to a target body part in a patient's body cavity, and imaging (capturing an image of) the target body part with the imaging unit 9. The image signal is transmitted and processed in the processor unit 26, and displayed as an endoscopic image on a display 19. The operating section 13 is also provided with a forceps inlet port 18 to insert a medical instrument. The forceps inlet port 18 is connected to a forceps tube 22.

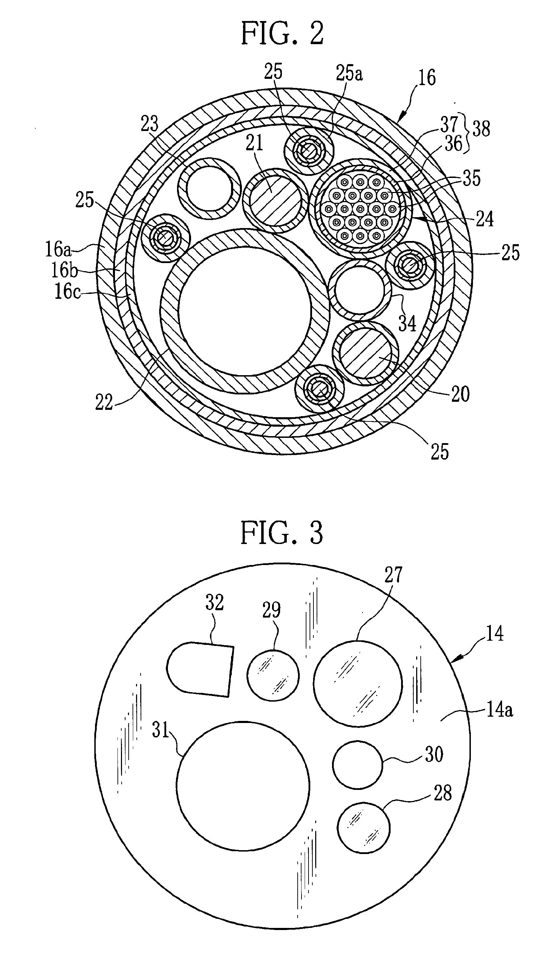

As shown in FIG. 2, the flexible tube part 16 has a three-layer structure composed of, from the inside to the outside, a spiral tube 16c called "flex" which provides flexibility while protecting the inside thereof, a net 16b called "blade" which surrounds the spiral tube 16c to prevent it from stretching, and an outer cover 16a of resin deposited on the net 16b. The flexible tube part 16 loosely surrounds all the elements including a pair of light guides 20, 21 for delivering illumination light to an illumination lens in the front end part 14, an air/water tube 23, a signal cable 24 and a jet-spray tube 34. A reference numeral 25 denotes the angle wire that is covered by a contact coil pipe 25a and operated to bend the curving part 15.

The signal cable 24 transmits a drive signal produced by the imaging controller in the processor unit 26 to the imaging unit 9, and also transmits an image signal of the target body part from the imaging unit 9 to the image processor in the processor unit 26. The signal cable 24 is composed of a plurality of signal lines 35, a steel pipe 36 that surrounds and shields a bundle of signal lines 35, and an outer tube 37 that is made of an electrical insulating material and surrounds the steel pipe 36. The steel pipe 36 and the outer tube 37 constitute an outer jacket 38. Instead of using the steel pipe 36 to surround the signal lines 35, it is possible to wrap a metal foil tape spirally around the signal lines 35. The signal line 35 is an electric insulated wire that is composed of a conductor and an insulating cover material.

As shown in FIG. 3, an end surface 14a of the front end part 14 has an observation window 27, two illumination windows 28, 29, a jet-spray opening 30, a forceps outlet port 31, and the air/water nozzle 32. The observation window 27 exposes a part of an objective optical system 41 to transmit the image light of the target body part in the body cavity. Each of the illumination windows 28, 29 exposes a part of the illumination lens that irradiates the illumination light, delivered from the light source unit through one of the light guides 20, 21, onto the region-of-interest. The forceps outlet port 31 is connected to the forceps inlet port 18 in the operating section 13 through the forceps tube 22. The air/water nozzle 32 discharges cleaning water or air to rinse the observation window 27 and the like as a water/air feed button (not shown) is pressed in the operating section 13. The jet-spray opening 30 applies a spray of fluid, such as natural air or carbon dioxide gas supplied from the air supply unit, to the region-of-interest.

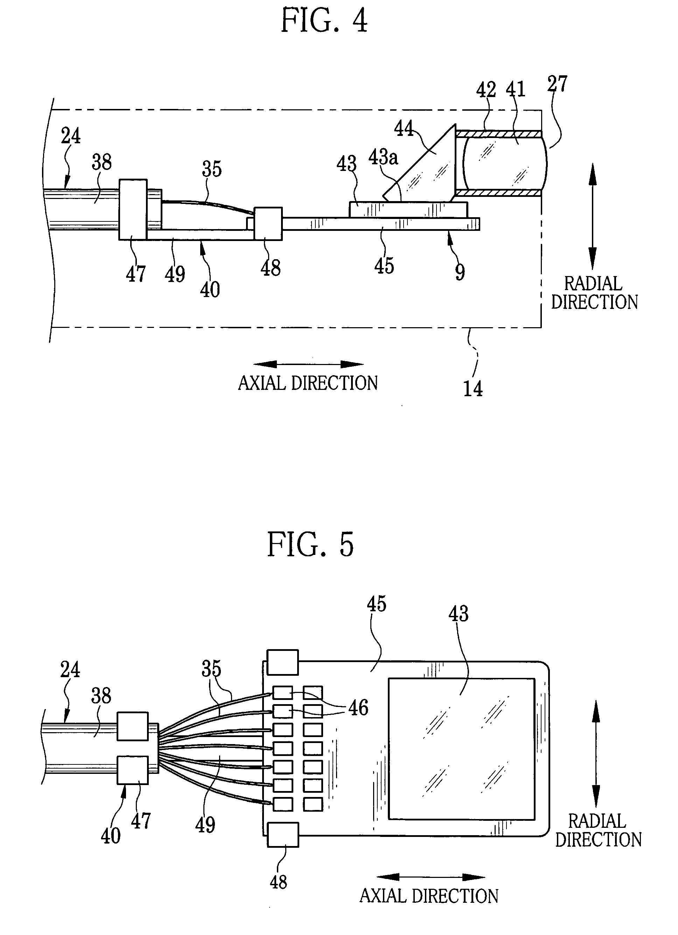

As shown in FIG. 4, the imaging unit 9, which is fixed inside the front end part 14, includes the objective optical system 41 to focus the image light of the region-of-interest through the observation window 27, a lens barrel 42 holding the objective optical system 41, a CCD image sensor (hereinafter, CCD) 43, a prism 44 for guiding the image light out of the objective optical system 41 to the CCD 43, and a circuit board 45 that mounts the CCD 43 and a CCD drive circuit. An optical axis of the objective optical system 41 extends parallel to an imaging surface 43a of the CCD 43. The circuit board 45 has a plurality of input/output terminals 46 (see, FIG. 5) electrically connected to the CCD 43 and the drive circuit.

As also shown in FIG. 5, the signal cable 24 comes close to a rear end of the imaging unit 9, and in front of the circuit board 45 the outer jacket 38 is removed to expose the signal lines 35, which are then soldered to the input/output terminals 46. The signal cable 24 is coupled to the circuit board 45 by the connector 40, with a reasonable distance to prevent tightening the signal lines 35.

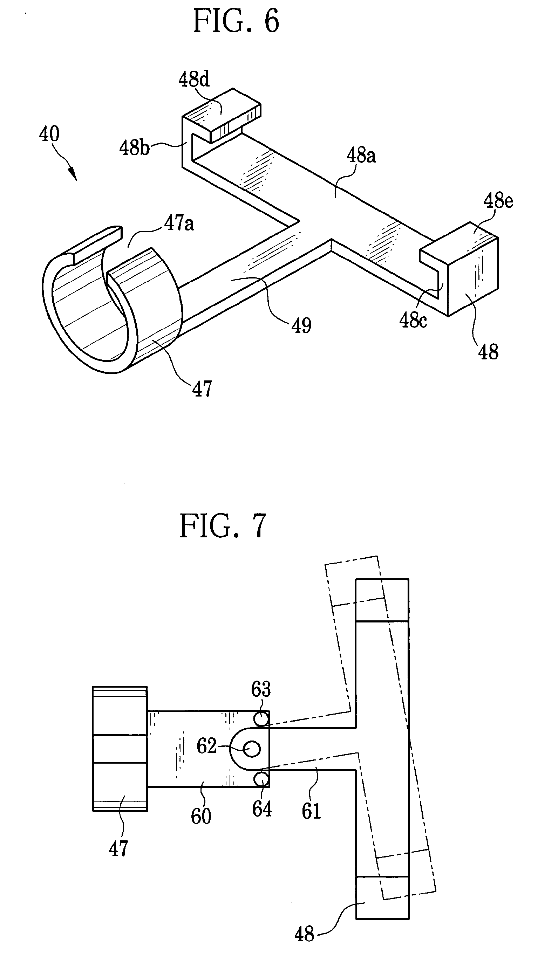

As better shown in FIG. 6, the connector 40 has a cable attachment ring (cable attachment portion) 47 that fits onto a front end portion of the signal cable 24, a circuit board attachment portion (imaging unit attachment portion) 48 that catches the circuit board 45, and a connecting pillar (connecting piece) 49 to connect the cable attachment ring 47 and the circuit board attachment part 48. The connector 40 is made of plastic having an electrical insulating property, and provides as much elasticity as it bends with a thrust of a finger.

An inner diameter of the cable attachment ring 47 is slightly smaller than an outer diameter of the signal cable 24. And the cable attachment ring 47 is disconnected in one place by a cut-out 47a. When attaching (fixing) the cable attachment ring 47 to the signal cable 24, the cut-out 47a is expanded with fingers, and the signal cable 24 is inserted through the cut-out 47a, and then the cut-out 47a is closed tightly. The cable attachment ring 47 is thereby fixed firmly to the front end portion of the signal cable 24.

The circuit board attachment part 48 includes a base plate 48a elongated in the width direction of the circuit board 45 so as to fit on a bottom surface thereof, two side plates 48b, 48c standing upright from both ends of the base plate 48a, and two retaining plates 48d, 48e vertically projecting from an upper interior surface of each of the side plates 48b, 48c. The side plates 48b, 48c are spaced apart by a distance substantially equal to the width of the circuit board 45, and hold the circuit board 45 from the width direction thereof. The retaining plates 48d, 48e extend parallel to the base plate 48a while keeping substantially the same distance to the base plate 48a as the thickness of the circuit board 45. Together with the base plate 48a, the retaining plates 48d, 48e hold the circuit board 45 from the thickness direction thereof. For better attachment strength, it is preferred to provide the both side edges of the circuit board 45 with a groove or such depression to fit onto the side plates 48b, 48c of the connector 40.

When attaching (fixing) the circuit board attachment part 48 to the circuit board 45, the retaining plates 48d, 48e are pulled away from each other with fingers, and the circuit board 45 is inserted between the retaining plates 48d, 48e. The connecting pillar 49 connects the center part in the width direction of the base plate 48a to the cable attachment ring 47.

The connector 40 serves to determine the relative position of the front end portion of the signal cable 24 to the circuit board 45, and provide an adequate strength in the connecting part of the signal cable 24 and the circuit board 45 to endure tensile load and torsional load.

Because of the detachability of the connector 40, the positional relationship between the signal cable 24 and the circuit board 45 can be adjusted easily during assembly of the electronic endoscope and such works. In the event of trouble, the connector 40 is simply removed and the signal cable 24 and the imaging unit 9 are poised for repair.

It may be possible to make the connector 40 swingable. As shown in FIG. 7, the cable attachment ring 47 is connected to a first link 60 instead of the connecting pillar 49, and the circuit board attachment part 48 is provided with a second link 61. Suspended by a rotary shaft 62, the second link 61 swings on the first link 60. On both sides of the rotary shaft 62, there are provided two stoppers 63, 64 which limit a swing angle of the second link 61. In the drawing, a chain double-dashed line illustrates the second link 61 swung all the way to the stopper 63. The rotary shaft 62 has a retaining member (not shown) on both ends. This configuration allows the cable attachment ring 47 and the circuit board attachment part 48 to move relative to each other on a plane parallel to the circuit board 45, and ensures smooth movement of the insertion section 11. It should be noted that the connector 40 may not be configured only to have one-degree of freedom as above described, but also to have two-degree of freedom.

Although the connector 40 in the above first embodiment is attached (fixed) to the circuit board 45, it may be attached to any spot of the imaging unit 9, such as the lens barrel 42.

### [Second Embodiment]

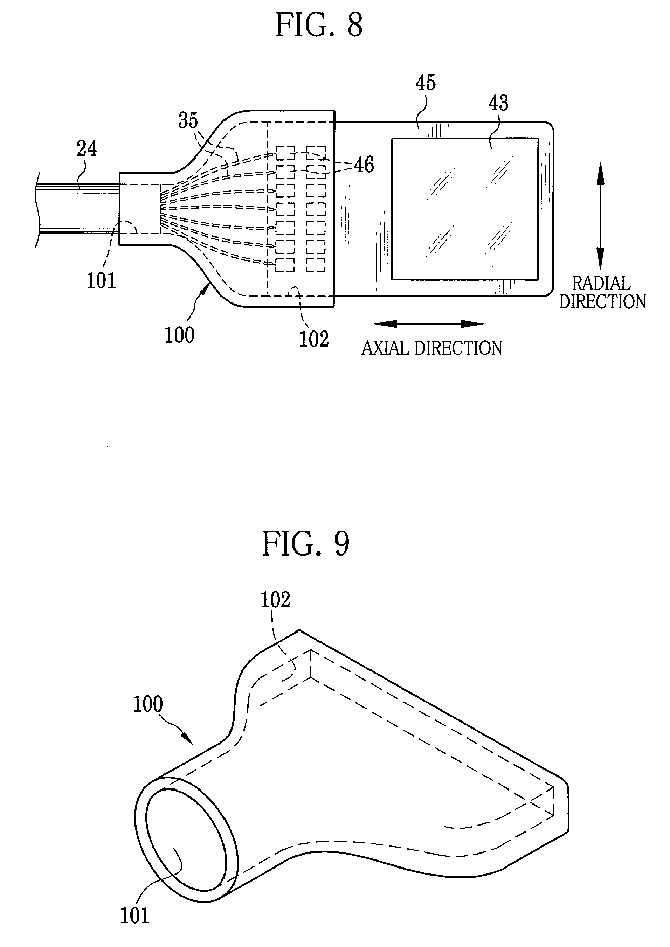

While the first embodiment is explained using the connector 40 which is composed of the cable attachment ring 47, the circuit board attachment part 48 and the connecting pillar 49, the connector may have a different configuration, such as a connector 100 shown in FIG. 8 and FIG. 9 that surrounds the signal lines 35 exposed from the signal cable 24 and the input/output terminals (contact terminals) 46. As with the first embodiment, it is also preferred that the connector 100 is fixed on one end to the front end portion of the signal cable 24 by a tightening force, and the other end to the circuit board 45 by a mechanical engagement, such as a groove or a kind of depression.

The connector 100 is made of rubber, and has a cable fitting part 101 to fit onto the front end portion of the signal cable 24, and a circuit board fitting part 102 to catch an end of the circuit board 45. Inside the connector 100, the cable fitting part 101 and the circuit board fitting part 102 communicate. Because of the elasticity of the connector 100, the cable fitting part 101 and the circuit board fitting part 102 are expanded with fingers, and put on or removed from the signal cable 24 and the circuit board 45. As well as having the same effect as the connector 40 of the first embodiment provides, the connector also provides an effect to protect the signal lines 35 exposed from the signal cable 24 and the input/output terminals 46.

Here, it should be noted that the connector may be made of plastic, instead of rubber. In this case, it is preferred to divide the connector 100 into two segments along the axial direction, and to provide one segment with an engaging claw and the other segment with an engaging hole, so as to prevent the connector 100 from easily disassembling. This configuration facilitates attaching and removing the connector 100 from the signal cable 24 and the circuit board 45.

Although the present invention has been fully described by the way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An electronic endoscope (10) **characterized in** comprising:
an insertion section (11) to be inserted into an object under observation;
an imaging unit (9) disposed at a front end of said insertion section (11), and for imaging a region-of-interest in said object;
a signal cable (24) extending through said insertion section (11) and composed of a plurality of signal lines (35) and a cable jacket (38) to surround said signal lines, said signal lines (35) being exposed from a front end of said signal cable (24) and connected to said imaging unit; and
a connector (40, 100) made of an electrical insulating material, and for connecting a front end portion of said signal cable (24) and said imaging unit (9).

2. The electronic endoscope of claim 1, wherein said connector (40, 100) is **characterized in** including:
a cable attachment part (47, 101) detachably attached to said front end portion;
an imaging unit attachment part (48, 102) detachably attached to said imaging unit (9); and
a connecting piece for connecting said cable attachment part (47) and said imaging unit attachment part (48).

3. The electronic endoscope of claim 1, wherein said connector (40, 100) surrounds the exposed part of said signal lines (35) and a plurality of contact terminals (46) which are located in said imaging device and connected to said signal lines.

4. The electronic endoscope of claim 3, wherein said connector (40, 100) is made of rubber.
